(19) Europäisches Patentamt
European Patent Office
Office européen des brevets

(11) Publication number: **0 448 848 A1**

(12) **EUROPEAN PATENT APPLICATION**

(21) Application number: **90203254.9**

(22) Date of filing: **11.12.90**

(51) Int. Cl.$^5$: **C07C 29/16**, C07C 27/22, C07C 45/50

The title of the invention has been amended (Guidelines for Examination in the EPO, A-III, 7.3).

A request for correction of the values of the tables within examples 7,8 and 9 has been filed pursuant to Rule 88 EPC. A decision on the request will be taken during the proceedings before the Examining Division (Guidelines for Examination in the EPO, A-V, 2.2).

Amended claims in accordance with Rule 86 (2) EPC.

(30) Priority: **12.12.89 IT 2266189**

(43) Date of publication of application:
**02.10.91 Bulletin 91/40**

(84) Designated Contracting States:
**AT BE CH DE DK ES FR GB GR LI LU NL SE**

(71) Applicant: **ENIRICERCHE S.p.A.**
**Corso Venezia 16**
**I-20121 Milan(IT)**

(72) Inventor: **Corain, Benedetto**
**Via Miazzo 24**
**I-35030 Selvazzano Dentro, Padua(IT)**
Inventor: **Basato, Marino**
**Via Miazzo 24**
**I-35030 Selvazzano Dentro, Padua(IT)**
Inventor: **Zecca, Marco**
**Via Montesuello 14**
**I-25128 Brescia(IT)**
Inventor: **Braca, Giuseppe**
**Viale delle Piagge 8**
**I-56100 Pisa(IT)**
Inventor: **Raspolli, Galletti Anne Maria**
**Via Meliani 56**
**I-56021 Cascina-Localita, Titignano-Pisa(IT)**
Inventor: **Lora, Silvano**
**Via Valvasori 1**
**I-35124 Padua(IT)**
Inventor: **Palma, Giancarlo**
**Via Cappuccina 38**
**I-30172 Venezia, Mestre(IT)**
Inventor: **Brunelli, Maurizio**
**Via Agadir 10/C**
**I-20097 San Donato Milanese, Milan(IT)**

(74) Representative: **Roggero, Sergio et al**
**Ing. Barzanò & Zanardo Milano S.p.A. Via Borgonuovo 10**
**I-20121 Milano(IT)**

(54) **Hydroformylation of olefins.**

(57) Process for direct conversicn of olefines to alcohols with an extra carbon atom compared with the olefine, by way of contact of an olefine, carbon monoxide and hydrogen with a catalyst suspended in an inert organic liquid means, whereby the catalyst may be defined as follows:
(P-NC)($RhL_3$)
formed by the association (adduced formation = adduct) of a complex of rhodium ($RhL_3$) and an isonitryl function (P-NC) solid polymer, obtaining the polymer by copolymerisation of an omega-isocyano alkyl (C1-C1O) acrylate or metacrylate with a (met)acrylic or vinylic conomomer, in the presence of a polyfunctional reticulating monomer, or dehydrating the formamide precursor polymer obtained by way of copolymerisation of an alkyl formamide (C1-C10) acrylate or metacrylate with a (met)acrylic and/or vinylic comonomer, in the presence of the polyfunctional cross-linking monomer. The use of this catalyst allows the conversion of olefines to alcohols in a single operational stage and does not involve hydrogenation of the olefine, nor its isomerisation.

EP 0 448 848 A1

Process for direct synthesis of olefine alcohols, carbon monoxide and hydrogen, promoted by a supported rhodium catalyst

The present invention concerns a catalytic process for the production of alcohols from olefines, carbon monoxide and hydrogen.

Hydroformylation or oxosynthesis, constitutes an important industrial process for the production of aldehydes and/or alcohols from olefines, carbon monoxide and hydrogen. Hydroformylation products are widely used raw materials for various industries. Hydroformilation catalysts used generally for the purpose, are based on cobalt or rhodium, more particularly rhodium complete with an organic bond. For details of the known technique reference should be made more particularly to the description in "New Synthesis with Carbon Monoxide", published by J. Falbe, Springer-Verlag, Berlin, Heidelberg, New York 1980, Chapter I. In the hydroformylation processes carried out in the organic homogeneous phase with oleo-soluble catalysts, the separation and recovery of the catalyst from the hydroformylation reaction mixture are difficult and have a strong incidence on production costs. A solution advanced to solve this problem consists in the hydroformylation reaction ensured in a mixed aqueous-organic environment, in the presence of an aqueous solution of rhodium hydrosoluble complex catalyst. As an example reference is made to the use of complexes containing rhodium and a hydrosoluble sulphonated triaryl phosphine as described in US Patent 4 248 802. In pursuing this process, at the end of the hydroformylation reaction it is possible to separate an organic phase containing the reaction products of an aqueous phase containing the catalyst. According to another known technique, described in French Patent 2 489 308, a mixed aqueous-organic system is used, incorporating into the reaction mixture a reagent with an affinity for the aqueous phase and the organic phase or a surface tension agent. However it has been found that operations in an mixed aqueous-organic environment normally calls for the use of high pressures and extended reaction times, particularly in the hydroformylation of higher olefines, and these conditions are of little interest from the industrial viewpoint. Finally a solution based on the use of adducts of metal complexes and insoluble materials has been evaluated over recent years as described in "Supported Metal Complexes", by F.R.Hartley, D Reidel Publishing Company, Dordrecht 1985, Chapter 8, though without producing industrially relevant results.

It has now been found according to the present invention, that adducts consisting of rhodium complexes with organic polymers, comprising isonitriles pendent groups, form active heterogenous catalysts in aldehyde olefine hydroformylation processes and in the subsequent reduction of aldehydes in alcohol, conducted under bland conditions in an organic environment. It has been found that these catalysts, in addition to being easily separated from the hydroformylation mixture owing to their heterogeneous nature, maintain an extended catalytic activity and may thus be re-used during further cycles. In accordance with this the present invention relates to a

process for direct olefine conversion into alcohols with an extra carbon atom compared with the olefine, by contact with an olefine, carbon monoxide and hydrogen with a catalyst suspended in inert organic liquid means, the said catalyst being defined by this formula:

$(P\text{-}NC)(RhL_3)$

and formed by the association (adduced formation = adduct) of:

- a rhodium complex RhL3 (L = bond); and
- a solid polymer with isonitrile function (P-NC), containing isonitrile groups of 0,1 to 1,5 meq/g, in which the -NC groups are present at the end of lateral pendent chains, the polymer being obtained by copolymerisation of an omega-isocyano alkyl (C1-C10) acrylate or metacrylate with acrylic and/or metacrylic and/or vinylic comonomer, in the presence of a polyfunctional cross-linking monomer, or by dehydration of the formamide precursor polymer produced by copolymerisation of a formamide alkyl (C1-C10) acrylate or metacrylate with an acrylic and/or metacrylic and/or vinylic comonomer, in the presence of a polyfunctional cross-linking monomer. According to this invention particularly useful $RhL_3$ rhodium complex in the preparation of the catalyst is $[Rh(CO)_3(acetylacetonate)]$.

The polymers used in the preparation of catalysts in this invention, are the products of polymerisation of at least one omega-isocyano alkyl (C1-C10) acrylate or metacrylate, or their formamide precursor (with successive dehydration), with an acrylic and/or metacrylic and/or vinylic comonomer, in the presence of a polyfunctional cross-linking monomer.

The alkyl part of the omega-isocyano alkyl acrylate or metacrylate contains 2 to 5 carbon atoms. Examples of omega-isocyano alkyl acrylate or metacrylate are 3-isocyano-propyl acrylate and metacrylate. Examples of formamide precursors are 3-formamide propyl acrylate and metacrylate.

The polyfunctional cross-linking comonomers useful in the preparation of the isonitrile polymer, are diacryloyl amide alkanes, and monomers with two or more functional acrylic or metacrylic groups. Those

containing 1 to 6 carbon atoms in the alkane part are particularly useful among the diacryloyl amide alkanes. Examples of diacryloyl amide alkanes are diacryloylamide methane (methylene biacrylamide) and diacryloyl amide hexane (hexamethylene biacrylamide). Among the polyfunctional acrylates and metacrylates, the di(met)acrylate and tri(met)acrylate polyoxyalkylene polyols are particularly useful, such as triethylene glycol dimetacrylate and trimethylol propane trimetacrylate. Preparation of the isonitrile function polymer also uses auxiliary (met)-acrylic and/or vinylic comonomers normally selected among methyl acrylate and metacrylate, dimethyl acrylamide, methoxy triethylene glycol metacrylate and acrilonitrile.

The isonitrile polymer is prepared by radical polymerisation of the monomers, catalysed by peroxides or ionising radiation (x-ray and gamma-ray), the general polymerisation conditions being the following.

A mixture of monomers is prepared, in which an omega-isocyano alkyl acrylate or metacrylate monomer is present in proportions of 5-25% by weight, a polyfunctional monomer such as a reticulator in proportions of 2-8% by weight and one or more among the acrylic, metacrylic or vinylic comonomers up to 100% by weight. The mixture is polymerised with radical polymerisation initiators, such as azobisisobutirronitrile, benzoyl peroxide or sodium persulphate, at normal polymerisation temperatures of 40° C-70° C, or with X-rays or gamma-rays working within a temperature range of -80 to 100° C preferably to convert at least 50% of the monomers. Residual monomers are removed from the polymerised material by washing with organic solvent, the polymer then being granulated.

According to a particular form of processing a mixture of monomers and water in the proportions indicated above is prepared. The mixture is injected drop by drop with a syringe into an excess of low boiling point hydrocarbon, such as petroleum ether, then maintained at a temperature between -30° C and the hydrocarbon solidification temperature. The system kept at low temperature is gamma-ray irradiated with Co-60 at such input speeds and radiation times as to ensure monomer conversion rates in excess of 50%. This process has the essential advantage of producing a highly porous granular polymer.

If an omega-isocyano alkyl acrylate or metacrylate formamide precursor is used, the monomer proportions and other conditions indicated above are again used, and the polymer obtained at the end of polymerisation is subjected to processing to transform the formamide groups into isonitrile groups. This processing is suitably conduoted by reinflating the formamide polymer with an excess of liquid and anhydrous organic nitrogen base (such as pyridine), adding and stirring in a suitable dehydration agent, such as p-toluene sulphonic acid chloride,

The isonitrile polymer thus obtained has an isonitrile groups content of 0.1 to 2.0 meq/g and is conveniently used in granular form of 0.1 to 2.0mm mesh-size.

The catalyst for the process according to the invention is obtained by association (adduct) of the $RhL_3$ rhodium complex with the isonitrile polymer (P-NC). This is usually achieved by dissolving the rhodium complex in a polar and aprotic organic solvent, viz.dichloromethane or dichloroethane, mixing the solution thus obtained with a suspension of the polymer (P-NC) in the same solvent. At the end of the formation reaction, the product is recovered by filtration, washed with suitable solvents and dried. The catalyst thus obtained is in granular form of similar mesh size to that of the support and its rhodium content will generally between 0.1 and 6.0% by weight.

The catalyst is useful as an heterogenous catalyst for processes of olefine hydroformylation and hydrogenation of the aldehydes thus obtained from the relevant alcohols. Olefines allowing hydroformylation are alpha-, internal-, linear or ramified olefines, usually with 2 to 16 carbon atoms and preferably 2 to 13 carbon atoms in the molecule. Such olefines may be n-butene, n-esene and e-decene.

The reaction is achieved by feeding the olefine and a mixture of hydrogen and carbon monoxide to the catalyst in suspension in an inert organic agent, operating at a temperature of 40° C to 140° C and at a pressure of 0.1 to 15 Mpa. Temperature and pressure valued will preferably vary betueen 60° and 120° C and 3 to 12 Mpa respectively. The inert solvent reaction agent is best selected among the solvents of polar aprotic aromatic nature and moderately polar aprotic nature. Specific examples of such useful solvents are toluene, benzene, xylenes, dichloroethane, products of hydroformylation and by-products of hydrocarbons and hydroformylation process top and tail ethers, recovered after rectification separation of aldehydes and alcohols. The molar ratio of input hydrogen and carbon monoxide may generally vary between 2/1 and 0,5/1 - preferably being around 1/1. Under such conditions the reaction duration for practically complete conversion of the olefine are of the order of 0,5 - 24 hours. By working in this manner, at the end of the reaction a mixture is obtained containing the main alcohol products with an extra carbon atom compared with supply olefine and the aldehyde secondary products.

The main advantages relating to the catalyst and process according to the present invention are:

- high catalytic activity maintained in subsequent recycling of the catalyst
- easy separation and re-use of catalyst in subsequent cycles;

- olefines hydroformylation into aldehydes and hydrogenation of the latter into alcohols in a single reaction phase;
- absence of hydrogenation and isomeriasation of olefine;
- relatively mild reaction condition; and
- possibility of continuous process operation.

The following experimental examples give a better illustration of the present invention.

Example 1

Preparation of isonitrile polymer (P1-NC)

A formamide precursor is prepared by copolymerisation, induced by radiation of the following monomers N,N'dimethyl acrylamide, 3-formamide propyl acrylate and methylene bi-acrylamide, the latter serving as the cross-linking agent. Copolymerisation is effected at low temperature (-78° C) in the presence of water, in order to produce a highly porous copolymer. In particular, a mixture containing 30% by weight of N,N'di-methyl acrylamide, 8% by weight of 3-formamide propyl acrylate, 2% by weight of methylene bi-acrylamide and 60% by weight of water is injected drop by drop with a syringe into a petroleum ether excess cooled to -78° C. This leads to the formation of vitreous particles with a diameter around 1,5 mm in which the water separates as small ice-crystals uniformly spread throughout the vitreous monomer phase. The petroleum ether suspension of these pearl-like particles is then polymerised until around 90% of the monomers have been converted, by gamma-ray radiation with Co-60 for 15 hours at 0.5 Gy/sec, and at -78° C. The resulting polymer pearls are then washed with water, ethanol and ethyl ether, in that order, and finally vacuum dried. For the purpose of scanner electron microscope (SEM) examination, some of the pearls expanded with water are frozen in liquid nitrogen, crushed and freeze-dried. Analysis on Cambridge Stereoscan 250 equipment reveals a highly porous structure.

The formamide precursor thus obtained is converted into the corresponding isonitrile polymer by adding p-toluene sulphonyl chloride to the polymer expanded with an excess of pyridine; complete generation of the isonitrile groups takes place within around 10 minutes. The analysis (carried out as described by R. ARSHADY et al, in Talanta, 31, 10A '1984'842) shows in particular an isonitrile groups content of 0,80 meq/g, and the infra-red spectrum shows the band of isonitrile groups at 2150 $cm^{-1}$, basic analyses revealing the following contents results: carbon 56,4%, hydrogen 8.9% and nitrogen 10.7% by weight. Repeat SEM analyses on the isonitrile polymer show that the highly porous structure of the formamide precursor is maintained.

The isonitrile polymer (P1-CN) thus obtained may be graphically reproduced as follows:

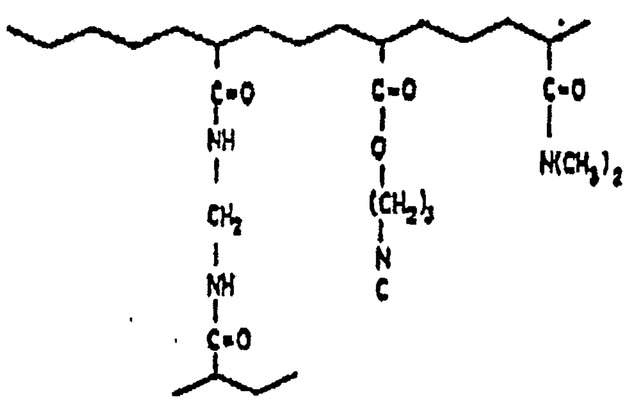

Example 2

Preparation of isonitrile polymer (P2-NC).

A mixture of methoxy triethylene glycol metacrylate (40% by weight), 3-formamide propyl acrylatc (16% by weight), triethylene glycol dimetacrylate (6.5% by weight) and water (37.5% by weight) is polymerised under the conditions as in Example 1. The monomer conversion rate is around 95%. This prepared formamide precursor is dehydrated as described in Example 1. An isonitrile polymer is thus produced in the

form of 1-2mm diameter pearls with an iso-nitrile groups content of 1.1 meq/g, with an infra-red spectrum showing the isonitrile group band at 2145cm[1]. Basic analyses show carbon contents 55.1%, nitrogen 2.0% and hydrogen 8.8% by weight.

Example 3

Preparation of isonitrile polymer (P3-NC).

A mixture of methoxy triethylene glycol metacrylate (36.5% by weight), 3-isocyano propyl acrylate (14.5% by weight), tri-ethylene glycol dimetacrylate (6.5% by weight) and water (42.5% by weight) is polymerised under the conditions in Example 1. The monomer conversion rate is around 50% and an isonitrile polymer is produced as 1-2mm diameter pearls with an isonitrile-groups content of 1.3 meq/g, with an infra-red spectrum showing a 2145 $cm^{-1}$ band and only traces of $=C=N=R$ groups (imminic groups) at 1635$cm^{-1}$. Basic analyses show carbon contents of 67.3%, nitrogen 1.8% and hydrogen 8.4% by weight.

The structure of isonitrile polymers (P2-NC) and (P3-NC) obtained as described in the Examples 2 and 3 may be graphically illustred in the following manner:

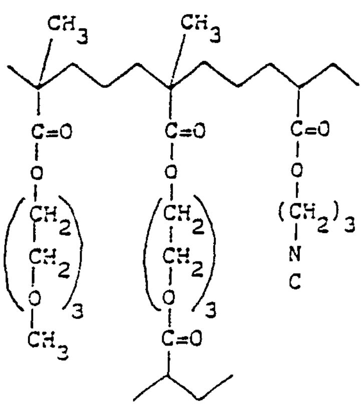

Example 4

Preparation of (P1-NC) [$Rh(acac)(CO)_2$] catalyst.

The complex of rhodium dicarbonyl acetyl acetonate [$Rh(acac)(CO)_2$] is prepared according to the method described by Y S VARSHAVSKII et Al., Russ. J. Inorg. Chem 12 6 (1969)899, and recrystallised with n-hexane. 0,195 g of rodium dicarbonyl acetyl acetonate dissolved in around 2.5 ml of 1.2-dichloroethane are added to the isonitrile polymer (P1-NC) (viz. 0.942 g in dry state) and expanded with the same solvent. The initial molar ratio of rhodium/isonitrile used is 1/1. After stirring gently for 2 hours, during which a certain amount of gas is developed, the catalyst is recovered by filtering, then washed with 1.2-dichloroethane (2 × 30 ml), acetone (3 × 30 ml) and ethyl ether (3 × 30 ml). The catalyst is vacuum-dried at ambient temperature for 2 hours. The rhodium content by weight of the catalyst thus obtained equals 5.0%; this represents a yield around 63% of the initial amount used for the reaction. The infra-red spectrum shows two symmetrical high intensity bands at 2184 $cm^{-1}$ (isonitrile group) and 1994 $cm^{-1}$ (carbonyl group); in addition there is no sign of non-coordinated isonitrile groups in the infra-red spectrum.

Example 5

Preparation of (P2-NC) [$Rh(acac)(CO)_2$] catalyst.

The procedure is practically as in Example 4. The isonitrile polymer used here is (P2-NC) instead of (P1-NC); the reagents contact time is also extended to 14 hours. This gives a catalyst containing 5.9% by weight of rhodium. This is a yield of around 60% of the initial reagents used. The infra-red spectrum shows two symmetrical high intensity bands at 2183 $cm^{-1}$ (isonitrile group band) and 1991 $cm^{-1}$ (carbonyl group band); there is no sign of non-coordinated isonitrile groups in the infra-red spectrum.

Example 6

Preparation of (P3-NC) [Rh(scac)$(CO)_2$] catalyst.

The adopted procedure is almost entirely as described in example 4. The isonitrile polymer used here is (P3-NC) instead of (P1-NC). The catalyst obtained here has a similar weight of rhodium as in examples 4 and 5. The yield here is around 55% of the initial reagents used. The infra-red spectrum shows two symmetrical high intensity bands at 2184$cm^{-1}$ (isonitrile group) and 1991$cm^{-1}$ (carbonyl band) and a low intensity band of 2335$cm^{-1}$; furthermore the infra-red spectrum shows no sign of non-coordinated isonitrile groups.

Example 7

The catalyst as described in example 4 is used for a 1-hexene hydroformylation test. The test is effected in a glass flask placed in a 150 ml tilting autoclave. This arrangement allows contact between reagents and catalyst while avoiding their dispersion in the autoclave, thus preventing system contact with autoclave walls. The latter is heated in an oil-bath. The hydroformylation reaction takes place at 120°C under hydrogen and carbon monoxide pressure of 12 MPa, with a molar ratio of 1:1. The solvent is toluene, the 1-hexene concentration being at 3 mol/dmc (3M) The test lasts 5 hours. The first cycle of hydroformylation is followed by two further cycles in the same conditions indicated above, with direct catalyst recirculation. Qualitative and quantitative analysis of reaction products is effected by gas chromatography/mass spectrometry (gas-mass, GCMS). Each cycle provides a practically complete conversion of 1-hexene into the following reaction products:

| Cycle | I | II | III |
|---|---|---|---|
| n-heptanal | 0,06 | 0,26 | 0,20 |
| 2-methyl hexanal | 0,11 | 0,29 | 0,29 |
| 2-ethyl penthanal | 0,07 | 0,11 | 0,10 |
| n-heptanol | 0,39 | 0,17 | 0,18 |
| 2-ethyl penthanol + 2-methyl hexanol | 0,36 | 0,13 | 0,13 |
| N/B * | 1,1 | 1,1 | 1,2 |
| selectivity (**) | 0,75 | 0,32 | 0,33 |
| Rh loss | 4% | 2% | <0,1% |
| olefine conversion | 100% | 100% | 100% |

(*) molar ratio between linear and ramified alcohols

(**) molar ratio between total alchols and converted 1-hexene

Example 8

The procedure in example 7 is followed, using the catalyst obtained as in example 5. Results are shown below.

| Cycle | I | II | III |
| --- | --- | --- | --- |
| n-heptanal | 0,12 | 0,31 | <0,1 |
| 2-methyl hexanal | 0,11 | 0,25 | <0,1 |
| 2-ethyl penthanal | 0,06 | 0,10 | <0,1 |
| n-heptanol | 0,38 | 0,20 | 0,52 |
| 2-ethyl penthanol + 2-methyl hexanol | 0,33 | 0,14 | 0,48 |
| N/B * | 1,0 | 1,0 | 1,1 |
| selectivity (**) | 0,71 | 0,34 | 1,0 |
| Rh loss | 3% | 2% | 1% |
| olefine conversion | 100% | 100% | 100% |

(*) molar ratio of linear and ramified alcohols

(**) molar ratio of total alcohols and converted 1-hexene

Example 9

The procedure in example 7 is followed, using the catalyst obtained as in example 6. Results are shown below.

| Cycle | I | II | III |
| --- | --- | --- | --- |
| n-heptanal | -- | 0,18 | 0,12 |
| 2-methyl hexanal | -- | 0,23 | 0.17 |
| 2-ethyl penthanal | -- | 0,11 | 0,09 |
| n-heptanol | 0,46 | 0,25 | 0,33 |
| 2-ethyl penthanol + 2-methyl hexanol | 0,54 | 0,23 | 0,20 |

| | | | |
|---|---|---|---|
| N/B (*) | 0,9 | 1,1 | 1,1 |
| selectivity (**) | 1,0 | 0,48 | 0,62 |
| Rh loss | 4,4% | 2,0% | 1,6% |
| olefine conversion | 100% | 100% | 100% |

(*) molar ratio of linear and ramified alcohols

(**) molar ratio of total alcohols and converted 1-hexene

## Claims

1. Process for direct conversion of olefines to alcohols with an extra carbon atom compared with the olefine, characterised in that, an olefine, carbon monoxide and hydrogen are brought into contact with a catalyst suspended in an inert organic liquid agent, the said catalyst being defined by the following formula:
(P-NC) ($RhL_3$)
and is formed by association (formation of an adduct) of:
   - a Rhodium $RHL_3$ complex (L = link); and
   - a solid polymer with an isonitrile function (P-NC), having an isonitriles group content of 0.1 to 1.5 meq/g, in which the -NC groups are present at the ends of pendent lateral chains, the said polymer being obtained by copolymerisation of an omega-isocyano alkyl (C1-C10) acrylate or metacrylate with an acrylic and/or metacrylic and/or vinylic comonomer, in the presence of a polyfunctional reticulating monomer, or by dehydration of the formamide precursor polymer obtained by copolymerisation of a formamide alkyl (C1-C10) acrylate or metacrylate with an acrylic and/or metalcrylic and/or vinylic comonomer, in the presence of the polyfunctional cross-linking monomer.

2. Process according to claim 1, characterised in that, the $RhL_3$ complex in the catalyst is [$Rh(CO)_2$acetyl acetonate)].

3. Process according to claim 1, characterised in that, the omega-isocyano acrylate or metacrylate contains 2 to 5 atoms of carbon in the alkyl part.

4. Process according to claim 3, characterised in that, the said omega-isocyano alkyl acrylate or metacrylate is selected from 3-isocyano propyl acrylate and metacrylate.

5. Process according to claim 1, characterised in that, in the solid polymer the said formamide precursor is selected from 3-formamide propyl acrylate and metacrylate.

6. Process according to claim 1, characterised in that, in the solid polymer the polyfunctional cross-linking monomers are diacryloyl amide alkanes and monomers with two or more acrylic or metacrylic functional groups.

7. Process according to claim 6, characterised in that, the said diacryloyl amide alkanes contain 1 to 6 carbon atoms in the alkane part and are preferably selected from diacryloyl amide methane (methylene biacrylamide) and diacryloyl amide hexane (hexa-methylene biacrylamide).

8. Process according to claim 6, characterised in that, the said monomers with two or more acrylic or metacrylic functional groups are di(met)acrylate and tri(met)acrylate polyoxy alkylene polyols preferably selected from triethylene glycol dimetacrylate and trimethylol propane trimetacrylate.

9. Process according to claim 1, characterised in that, in the solid polymer the said met (acrylic) and/or vinylic comonomers are selected from methyl acrylate and metacrylate, dimethylacrylamide, methoxy

triethylene glycol metacrylate and acrylonitrile.

**10.** Process according to claim 1, characterised in that, the said solid polymer contains 5-25% by weight of omega-isocyano alkyl acrylate or metacrylate, 2-8% by weight of cross-linking poly-functional monomer, the remaining percentage consisting of one or several among the (met) acrylic or vinylic comonomers.

**11.** Process according to claim 1, characterised in that, the catalyst contains 0.1 to 6.0% by weight of Rhodium in the form of 0.1-2.0mm mesh granules.

**12.** Process according to claim 1, characterised in that, alpha-olefines or internal-, linear- or ramified olefines, containing 2 to 16 - preferably 2 to 13 - carbon atoms are made to react, and the said olefines together with a mixture of hydrogen and carbon monoxide are fed to the catalyst suspended in an inert organic agent, operating at a temperature of 40° C to 140° C and under a pressure of 0.1 to 15 MPa.

**13.** Process according to claim 12, characterised in that, the operation is carried out at a temperature of 60° C to 120° C and under a pressure of 3 to 12 MPa, with an inert polar aprotic aromatic solvent or moderately polar aprotic solvent selected from toluene, benzene, xylene, dichloroethane, reaction products of hydroformylation and hydrocarbon by-products and top & tail hydroformylation reaction ethers, recovered after rectification separation of aldehydes and alcohols, with a 2/1 or 5/1 - preferably 1/1 - molar ratio of hydrogen and carbon monoxide and for periods of 0.5 to 24 hours.

European Patent Office

# EUROPEAN SEARCH REPORT

Application Number

EP 90203254.9

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | US - A - 4 774 361 (JOHN M. MAHER et al.) * Claims 1,2 * -- | 1,5-7, 9,11, 13 | C 07 C 29/16 C 07 C 27/22 C 07 C 45/50 |
| Y | EP - A2 - 0 208 921 (HENKEL CORPORATION) * Abstract * -- | 1 | |
| Y | US - A - 4 214 109 (JOHN T. CARLOCK) * Claim 1 * -- | 1 | |
| Y | EP - A1 - 0 014 225 (THE DOW CHEMICAL) * Claims 1,2,4-6 * -- | 1 | |
| A | DE - A1 - 3 012 729 (UOP INC.) * Claim 1 * -- | 1 | |
| D,A | FR - B1 - 2 489 308 (JOHNSON MATTHEY) * Claims 1-7,11-13 * -- | 1 | TECHNICAL FIELDS SEARCHED (Int. Cl.5) |
| A | US - E - 31 812 (EMILE KUNTZ) * Claims 1,18-22 * | 1,12, 13 | C 07 C 27/00 C 07 C 29/00 C 07 C 31/00 C 07 C 45/00 C 07 C 47/00 B 01 J 31/00 |
| D,A | & US-A-4 248 802 -- | | |
| A | EP - A1 - 0 309 056 (SHELL INTERNATIONALE) * Abstract * -- | 1 | |
| A | US - A - 4 334 101 (ELVIO MANTOVANI et al.) * Abstract * ---- | 1 | |

The present search report has been drawn up for all claims

| Place of search | Date of completion of the search | Examiner |
|---|---|---|
| VIENNA | 01-03-1991 | REIF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)